# EUROPÄISCHE PATENTSCHRIFT

(11) **EP 2 935 198 B1**
(45) Veröffentlichungstag und Bekanntmachung des Hinweises auf die Patenterteilung: **05.06.2019**
(21) Anmeldenummer: 13801590.4
(22) Anmeldetag: 09.12.2013
(51) Int. Cl.: C07C 67/62, C07C 51/50

(54) **VERFAHREN ZUR STABILISIERUNG VON POLYMERISATIONSFÄHIGEN VERBINDUNGEN**
METHOD FOR STABILISING POLYMERISABLE COMPOUNDS
PROCÉDÉ DE STABILISATION DE COMPOSÉS APTES À LA POLYMÉRISATION

(30) Priorität: 19.12.2012 DE 102012223695; 19.12.2012 US 201261739019 P
(43) Veröffentlichungstag der Anmeldung: 28.10.2015
(73) Patentinhaber: BASF SE, 67056 Ludwigshafen am Rhein (DE)
(72) Erfinder: KORN, Tobias Johannes, 67061 Ludwigshafen (DE); ZUROWSKI, Peter, 76829 Landau (DE); FRIESE, Thorsten, 68199 Mannheim (DE); HAREMZA, Sylke, 69151 Neckargemünd (DE); JÄGER, Ulrich, 67354 Römerberg (DE); RISSEL, Steffen, 67281 Kirchheim (DE); SCHLIEPHAKE, Volker, 67105 Schifferstadt (DE)
(86) Internationale Anmeldenummer: PCT/EP2013/075875
(87) Internationale Veröffentlichungsnummer: WO 2014/095437

(56) Entgegenhaltungen:
- JP-A- 2001 348 359
- US-A- 4 507 495

## Beschreibung

Die Erfindung betrifft ein Verfahren zur Stabilisierung von (Meth)acrylsäure und (Meth)acrylsäureestern in flüssiger Phase durch Zusatz eines Cu-, Mn- oder Ce-haltigen Polymerisationsinhibitors, die bei der Ausübung des Verfahrens erzeugten flüssigen Phasen und die Verwendung des Cu-, Mn- oder Ce-haltigen Polymerisationsinhibitors zur Stabilisierung von (Meth)acrylsäure und (Meth)acrylsäureestern in flüssiger Phase.

Acrylsäure, Methacrylsäure und deren Ester sind bedeutende Monomere zur Erzeugung von Polymerisaten, die z.B. als Klebstoffe oder als superabsorbierende Materialien eingesetzt werden. Ein großes Problem bei der Herstellung und Umsetzung dieser Verbindungen stellt jedoch die hohe spontane Polymerisationsneigung aufgrund ihrer reaktiven Doppelbindungen dar.

Im Folgenden werden Acrylsäure und/oder Methacrylsäure abkürzend (Meth)acrylsäure genannt, sowie deren jeweiligen Ester (Meth)acrylsäureester.

Es ist seit langem bekannt, dass durch Zusatz von Inhibitoren (auch Retarder genannt) zu in flüssiger Phase befindlicher (Meth)acrylsäure und deren Estern polymerisationsfördernden Einflüssen entgegengewirkt werden kann.

Die Vielfalt der diesbezüglich im Stand der Technik empfohlenen Inhibitoren ist nahezu unbegrenzt (vgl. z.B. EP 765 856 A und DE 69 701 590 T2, die einen kleinen Ausschnitt dieser Inhibitoren würdigen) und umfasst auch Verbindungen der Elemente Kupfer (vgl. z.B. JP-A 2001-348359), Mangan (vgl. z.B. US 4,814,493, US 4, 542,231, US 4, 507, 495) und Cer (vgl. z.B. WO 2002/035596 A).

Obwohl mit den bekannten Systemen schon gute Ergebnisse erzielt werden, vermag jedoch keiner der bekannten Inhibitoren vollständig zu befriedigen. So entstehen weiterhin hohe Kosten durch die in kurzen Abständen notwendige Reinigung der Produktionsanlagen, und die volle Kapazität der Anlagen kann nicht genutzt werden.

Aufgabe war es daher, weitere Polymerisationsinhibitoren zur Verfügung zu stellen, welche den bekannten Systemen zumindest in Teilaspekten überlegen sind und welche insbesondere die Polymerisationsneigung von (Meth)acrylsäure und deren Estern in flüssiger Phase wirksamer unterdrücken können.

Es wurde gefunden, dass sich Komplexe von Kupfer, Mangan und Cer mit speziellen stickstoffhaltigen Liganden in besonderer Weise als Polymerisationsinhibitoren für (Meth)acrylsäure und (Meth)acrylsäureester in flüssiger Phase eignen.

Gegenstand der Erfindung ist daher ein Verfahren zur Stabilisierung von Acrylverbindungen, wobei man eine flüssige Phase, welche mindestens eine Acrylverbindung aus der Gruppe Acrylsäure, Methacrylsäure und deren jeweiligen Estern enthält, mit mindestens einem Metall aus der Gruppe Kupfer, Mangan und Cer versetzt sowie mindestens einem Liganden aus der Gruppe bestehend aus
a) Chinolinverbindungen der Formel (I) 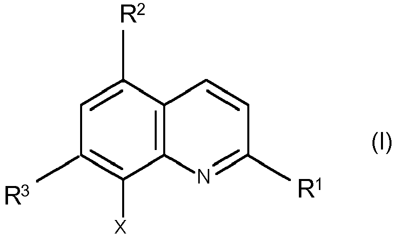 wobei die Symbole folgende Bedeutungen haben:
   - X: ist OH, NH₂, O-C₁-C₄-Alkyl, vorzugsweise OCH₃, O-C(O)-C₁-C₄-Alkyl, vorzugsweise O-C(O)-CH₃, O-C(O)-C₂H5 oder O-C(O)-Phenyl;
   - R¹: ist H, oder (C₁-C₄)-Alkyl, vorzugsweise Methyl;
   - R²: ist H, C₁-C₄-Alkyl, vorzugsweise Methyl, Cl, Br oder SO₃H und
   - R³: ist H, Cl oder Br;
   sowie N-Oxiden von Verbindungen der Formel (I),
b) 2,2'-Bis(2,3-dihydro-3-oxoindolyliden) und
c) aliphatischen y-zähnigen Liganden mit y = 2 - 6, enthaltend mindestens zwei Stickstoffatome und y-2 weitere koordinierende Stickstoff- oder Heteroatome enthaltende aliphatische oder aromatische C₁-C₄ Kohlenstoffbrücken verbunden sind, ausgewählt aus 1,1,4,7,10,10-Hexamethyltriethylentetramin, N.N.N',N",N"-Pentamethyl-diethylentriamin, N,N,N',N'-Tetramethylethan-1,2-diamin, N,N,N',N'-Tetramethylpropan-1,3-diamin, 1,3,5-Tris(dimethylaminopropyl)-sym-hexahydrotriazin, Bis(2-dimethylaminoethyl)ether und Tris[2-(dimethylamino)ethyl]amin.

Weiterhin Gegenstand der Erfindung ist eine flüssige Phase, enthaltend mindestens 10 Gew.-% an mindestens einer Verbindung aus der Gruppe Acrylsäure, Methacrylsäure und deren jeweiligen Estern und mindestens einem Komplex, enthaltend mindestens ein Metall aus der Gruppe Kupfer, Mangan und Cer sowie mindestens einen Liganden aus der Gruppe bestehend aus a) Chinolinverbindungen der Formel (I) sowie N-Oxiden von Verbindungen der Formel (I), b) 2,2'-Bis(2,3-dihydro-3-oxoindolyliden), und c) aliphatischen y-zähnigen Liganden mit y = 2 - 6, enthaltend mindestens zwei Stickstoffatome und y-2 weitere koordinierende Stickstoff- oder Heteroatome enthaltende aliphatische oder aromatische C₁-C₄ Kohlenstoffbrücken verbunden sind, ausgewählt aus 1,1,4,7,10,10-Hexamethyltriethylentetramin, N.N.N',N",N"-Pentamethyldiethylentriamin, N,N,N',N'-Tetramethylethan-1,2-diamin, N,N,N',N'-Tetramethylpropan-1,3-diamin, 1,3,5-Tris(dimethylaminopropyl)-sym-hexahydrotriazin, Bis(2-dimethylaminoethyl)ether und Tris[2-(dimethylamino)ethyl]amin.

Ebenfalls Gegenstand der Erfindung ist die Verwendung eines Komplexes, enthaltend mindestens ein Metall aus der Gruppe Kupfer, Mangan und Cer sowie mindestens einen Liganden aus der Gruppe bestehend aus a) Chinolinverbindungen der Formel (I) sowie N-Oxiden von Verbindungen der Formel (I), b) 2,2'-Bis(2,3-dihydro-3-oxoindolyliden), und c) aliphatischen y-zähnigen Liganden mit y = 2 - 6, enthaltend mindestens zwei Stickstoffatome und y-2 weitere koordinierende Stickstoff- oder Heteroatome enthaltende aliphatische oder aromatische C₁-C₄ Kohlenstoffbrücken verbunden sind, ausgewählt aus 1,1,4,7,10,10-Hexamethyltriethylentetramin, N.N.N',N",N"-Pentamethyl-diethylentriamin, N,N,N',N'-Tetramethylethan-1,2-diamin, N,N,N',N'-Tetramethylpropan-1,3-diamin, 1,3,5-Tris(dimethylaminopropyl)-sym-hexahydrotriazin, Bis(2-dimethylaminoethyl)ether und Tris[2-(dimethylamino)ethyl]amin, als Polymerisationsinhibitor zur Stabilisierung von Acrylsäure, Methacrylsäure und/oder deren jeweiligen Estern.

Die erfindungsgemäßen Polymerisationsinhibitoren zeigen insbesondere bei Acrylsäure und Methacrylsäure eine deutlich stärkere Inhibierung als bekannte Systeme wie Phenothiazin.

Der in dem erfindungsgemäßen Verfahren eingesetzte mindestens eine Ligand ist bevorzugt aus der Gruppe a) der Chinolinverbindungen der Formel (I) sowie N-Oxiden von Verbindungen der Formel (I), b) 2,2'-Bis(2,3-dihydro-3-oxoindolyliden), und c) aliphatischen y-zähnigen Liganden mit y = 2 - 6, enthaltend mindestens zwei Stickstoffatome und y-2 weitere koordinierende Stickstoff- oder Heteroatome enthaltende aliphatische oder aromatische C₁-C₄ Kohlenstoffbrücken verbunden sind. Es können auch beliebige Mischungen der erfindungsgemäßen Liganden verwendet werden.

Aus den Liganden der Gruppe a) sind solche Chinolinverbindungen der Formel (I) und deren N-Oxide bevorzugt, bei denen die Symbole folgende Bedeutungen haben:
- X: ist OH, NH₂, OCH₃, O-C(O)CH₃, O-C(O)C₂H₅ oder O-C(O)Phenyl;
- R¹: ist H oder CH₃;
- R²: ist H, CH₃, NH₂, Cl, Br oder SO₃H und
- R³: ist H oder Cl.

Besonders bevorzugt sind Chinolinverbindungen der Formel (I) und deren N-Oxide, bei denen die Symbole folgende Bedeutungen haben:
- X: ist OH oder NH₂;
- R¹: ist H oder CH₃;
- R²: ist H, CH₃, Cl oder NH₂ und
- R³: ist H.

Weiterhin besonders bevorzugt sind Chinolinverbindungen der Formel (I) und deren N-Oxide aus der Gruppe 8-Hydroxychinolin, 8-Hydroxy-5-methylchinolin, 5-Chlorchinolin-8-yl-propionat, 5-Chlor-8-hydroxychinolin, 5,7-Dibrom-8-hydroxy-2-methylchinolin, 8-Hydroxy-2-methylchinolin (8-Hydroxychinaldin), 8-Acetoxychinolin, 8-Aminochinolin, 8-Amino-2-methylchinolin (8-Aminochinaldin), 5-Amino-8-hydroxychinolin und 8-Hydroxychinolin-N-Oxid.

Ganz besonders bevorzugt sind Chinolinverbindungen der Formel (I) und deren N-Oxide aus der Gruppe 8-Hydroxychinolin, 5-Chlor-8-hydroxychinolin, 5-Amino-8-hydroxychinolin und 8-Hydroxychinolin-N-Oxid.

Insbesondere bevorzugt ist 8-Hydroxychinolin.

Ein ebenfalls bevorzugt Ligand ist die Gruppe b) mit 2,2'-Bis(2,3-dihydro-3-oxoindolyliden) (Indigo).

Ebenfalls bevorzugt sind Liganden der Gruppe c), die aus aliphatischen y-zähnigen Liganden mit y = 2 - 6, enthaltend mindestens zwei Stickstoffatome und y-2 weitere koordinierende Stickstoff- oder Heteroatome enthaltende aliphatische oder aromatische C₁-C₄ Kohlenstoffbrücken verbunden sind, ausgewählt aus 1,1,4,7,10,10-Hexamethyltriethylentetramin, N,N,N',N",N"-Pentamethyl-diethylentriamin (Lupragen® N301 der Fa. BASF SE, Ludwigshafen), N,N,N',N'-Tetramethylethan-1,2-diamin, N,N,N',N'-Tetramethylpropan-1,3-diamin, 1,3,5-Tris(dimethylaminopropyl)-sym-hexahydrotriazin (Lupragen® N600 der Fa. BASF SE, Ludwigshafen), Bis(2-dimethylaminoethyl)ether (Lupragen® N205 der Fa. BASF SE, Ludwigshafen) und Tris[2-(dimethylamino)ethyl]amin, besteht.

Besonders bevorzugt aus dieser Gruppe sind 1,1,4,7,10,10-Hexamethyltriethylentetramin, 1,3,5-Tris(dimethylaminopropyl)-sym-hexahydrotriazin (Lupragen® N600 der Fa. BASF SE, Ludwigshafen) und Bis(2-dimethylaminoethyl)ether (Lupragen® N205 der Fa. BASF SE, Ludwigshafen)

Erfindungsgemäß werden bevorzugt 0,01 mol-ppm bis 5 mol-%, besonders bevorzugt 0,1 mol ppm bis 3 mol-%, ganz besonders bevorzugt 0,5 mol-ppm bis 1 mol-%, insbesondere 2 mol-ppm bis 0,1 mol-% des Liganden, bezogen auf die Acrylverbindung, eingesetzt.

Erfindungsgemäß enthält der als Polymerisationsinhibitor eingesetzte Komplex mindestens ein, vorzugsweise genau ein Metall aus der Gruppe Kupfer, Mangan und Cer, insbesondere Kupfer(II), Kupfer(I), Mangan(II) und Cer(III). Bevorzugt sind Kupfer(II), Kupfer(I) und Mangan(II). Besonders bevorzugt sind Kupfer(II) und Kupfer(I). Ganz bevorzugt ist Kupfer(II).

Das Metall wird als Salz eingesetzt; geeignete Metallsalze sind z. B. Kupfer(II)phenolat, Kupfer(II)acetylacetonat, Kupfer(II)gluconat, Kupfer(II)tartrat, Kupfer(II)acetat, Kupfer(II)formiat, Kupfer(II)nitrat, Kupfer(II)hydroxid, Kupfer(II)sulfat, Kupfer(II)carbonat, Kupfer(II)naphthenat, Kupfer(II)acrylat, Kupfer(II)halogenide wie z.B. Kupfer(II)chlorid, Kupfer(II)salicylat, Kupfer(II)suIfonat, Kupfer(II)propionat, Kupfer(II)octanat, die jeweils auch Hydratwasser aufweisen können. Ferner eignen sich Kupfer(I)-verbindungen wie CuCI, CuCN, CuJ, CuBr, Cu(I)acetat, Cu₂SO₄, Cu₂O und CuCN, aber auch Salze von komplexen Kupfer(I)-anionen wie Cu(CN)₄³⁻ oder komplexen Kupfer(I)-kationen wie Cu(NH₃)₄⁺. Als Zusatz zu wässrigen flüssigen Phasen sind Kupfer(I)-salze weniger geeignet, da das Cu⁺ in selbigen zur Disproportionierung neigt Weiterhin geeignet sind die entsprechenden Mangan- und Cersalze. Cu(II)acetat ist besonders bevorzugt.

Prinzipiell kann die der flüssigen Phase zuzusetzende Verbindung des Metalls, insbesondere Kupfers in der flüssigen Phase in feinteiliger Form dispers verteilt werden (z.B. als feinteiliger Feststoff oder als dispers verteilte feinteilige Flüssigkeitströpfchen (gegebenenfalls einer die Metallverbindung enthaltenden Lösung)).

Erfindungsgemäß bevorzugt wird die der flüssigen Phase zuzusetzende Metall- insbesondere Kupferverbindung jedoch in der flüssigen Phase gelöst. Die flüssige Phase kann selbst eine Lösung oder eine flüssige Phase in einem aus mehreren flüssigen Phasen bestehenden System sein.

In einer bevorzugten Ausführungsform des erfindungsgemäßen Verfahrens wird der erfindungsgemäß als Polymerisationsinhibitor eingesetzte Komplex als solcher der flüssigen Phase zugesetzt.

In einer weiteren bevorzugten Ausführungsform des erfindungsgemäßen Verfahrens werden Metallsalz, insbesondere Kupfersalz, und Ligand der flüssigen Phase zugesetzt und der erfindungsgemäß als Polymerisationsinhibitor eingesetzte Komplex bildet sich in der flüssigen Phase.

Bezogen auf die in der flüssigen Phase enthaltene molare Menge an Acrylverbindung, insbesondere Acrylsäure wird man beim erfindungsgemäßen Verfahren das wenigstens eine Metall aus der Gruppe Cu, Mn und Ce in solchen Mengen zusetzen, dass der Gehalt G der flüssigen Phase an Metall, insbesondere Cu, bezogen auf die darin enthaltene molare Menge an Acrylverbindung, insbesondere Acrylsäure, in der Regel 0,01 mol-ppm bis 5 mol-% oder bis 3 mol-% beträgt. D.h., G kann beim erfindungsgemäßen Verfahren 0,05 mol-ppm bis 2 mol-%, oder 0,1 mol-ppm bis 1 mol-%, oder 1 mol-ppm bis 5000 mol-ppm, oder 3 mol-ppm bis 3000 mol-ppm, oder 5 mol-ppm bis 1000 mol-ppm, oder 10 mol-ppm bis 800 mol-ppm, oder 20 mol-ppm bis 500 mol-ppm, oder 30 mol-ppm bis 300 mol-ppm, oder 40 mol-ppm bis 200 mol-ppm, oder 50 mol-ppm bis 100 mol-ppm, oder 0,1 mol-ppm bis 10 mol-ppm betragen.

Das bevorzugte molare Verhältnis von Metall zu Ligand beträgt im Allgemeinen 1:1 bis 1:10, bevorzugt 1:1 bis 1:6, besonders bevorzugt 1:1 bis 1:4, ganz besonders bevorzugt 1:2.

Metallsalz und Ligand beziehungsweise der Komplex können der flüssigen Phase z.B. als Reinsubstanz oder vorzugsweise in Lösung zugesetzt werden. Als Lösungsmittel kann z.B. die flüssige Phase selbst oder die Acrylverbindung, insbesondere Acrylsäure (in der Regel eine erhöhte Reinheit aufweisende Acrylsäure), oder dasjenige Lösungsmittel, in welchem die Acrylverbindung, insbesondere Acrylsäure, in der flüssigen Phase gelöst ist, oder ein Bestandteil oder ein Gemisch aus mehreren Bestandteilen dieses Lösungsmittels verwendet werden. Wird der Komplex als Reinsalz zugesetzt, so können auch dessen polymorphe Formen eingesetzt werden. Als Polymorphie bezeichnet man die Fähigkeit von chemischen Verbindungen, bei gleicher Summenformel in verschiedenen Kristallstrukturen aufzutreten (s. Epple, Biometalle und Biomaterialien, Teubner-Verlag, 1. Auflage 2003, Seite 6). So können zum Beispiel vom Kupfer(II)-Komplex mit 8-Hydroxychinolin die α-, β-, und γ-form eingesetzt werden.

Bei den erfindungsgemäß stabilisierten Acrylverbindungen handelt es sich um(Meth)acrylsäure und Ester der beiden Säuren. Bevorzugt als Ester sind geradkettige oder verzweigte Alkylester, vorzugsweise mit 1 bis 20 Kohlenstoffatomen. Beispiele für bevorzugte Acrylate sind Methylacrylat, Ethylacrylat, n-Butylacrylat, tert.-Butylacrylat und 2-Ethylhexylacrylat Besonders bevorzugt sind Acrylsäure und Methacrylsäure. Acrylsäure ist insbesondere bevorzugt.

Häufig wird die flüssige Phase bei dem erfindungsgemäßen Verfahren wenigstens 20 Gew.-%, oder wenigstens 30 Gew.-%, oder wenigstens 40 Gew.-%, oder wenigstens 50 Gew.-%, oder wenigstens 60 Gew.-%, oder wenigstens 70 Gew.-%, oder wenigstens 80 Gew.-%, oder wenigstens 90 Gew.-%, oder wenigstens 95 Gew.-%, oder wenigstens 98 Gew.-% an Acrylverbindung enthalten (jeweils bezogen auf das Gewicht der flüssigen Phase)
Das erfindungsgemäße Verfahren eignet sich insbesondere zur Stabilisierung von Acrylsäure. Die Herstellung der Acrylsäure kann z.B. durch heterogen katalysierte partielle Oxidation einer C₃-Vorläuferverbindung (z.B. Propylen, Propan, Acrolein, Propionaldehyd, Propionsäure, Propanol und/oder Glyzerin) in der Gasphase erfolgen (vgl. z.B. WO 2010/012586, USA 5,198,578, EP-A 1 710 227, EP-A 1 015 410, EP-A 1 484303, EP-A 1 484 308, EP-A 1 484 309, US-A 2004/0242826, WO 2006/136336, DE-A 10 028 582 und WO 2007/074044).

Erfindungsgemäß werden die Polymerisationsinhibitoren aus Metall und Ligand bevorzugt an solchen Stellen eingesetzt, an denen die (Meth)acrylsäure oder deren jeweiligen Ester beispielsweise durch hohe Reinheit, hohe Verweilzeit und/oder hohe Temperatur einer Polymerisationsgefahr ausgesetzt sind. Das erfindungsgemäße Verfahren eignet sich daher sowohl zur Stabilisierung von in einer flüssigen Phase befindlicher (Meth)acrylsäure oder deren jeweiliger Estern während deren Lagerung als auch während deren prozessualen Handhabung. Der letztere Fall liegt insbesondere dann vor, wenn die flüssige Phase einem nicht kristallisativen thermischen Trennverfahren unterworfen wird (die dabei auftretenden Temperaturen liegen in der Regel oberhalb von 50°C, meist oberhalb von 60°C oder 70°C, oder oberhalb von 90°C oder 110°C). Dabei handelt es sich in der Regel um solche thermischen Trennverfahren, bei denen in trennwirksame Einbauten enthaltenden Trennkolonnen gasförmige (aufsteigend) und flüssige (absteigend) Stoffströme bzw. zwei flüssige Stoffströme im Gegenstrom geführt werden, wobei infolge der zwischen den Stoffströmen bestehenden Gradienten ein Wärme- und Stoffaustausch stattfindet, der letztlich die in der Trennkolonne erwünschte Trennwirkung bedingt. Beispiele für solche nicht kristallisative thermische Trennverfahren sind die Rektifikation, die azeotrope Rektifikation, die Extraktion, die Desorption, die Strippung, die Destillation, die Azeotropdestillation und die Adsorption.Selbstredend eignet sich das erfindungsgemäße Verfahren der Polymerisationsinhibierung auch dann, wenn die flüssige Phase einem kristallisativen thermischen Trennverfahren unterworfen wird.

Der Begriff "thermische Trennverfahren" soll dabei zum Ausdruck bringen, dass dem System zur Erzielung der gewünschten Trennwirkung Wärme zugeführt oder entzogen werden muss (vgl. DE-A 10 2008 041573 und DE-A 10 2008 8040799).

Die prozessual zu behandelnde flüssige Phase kann dabei die erfindungsgemäß zuzusetzende, wenigstens eine Komplexverbindung bereits von Beginn des thermischen Trennverfahrens an zugesetzt enthalten (d.h., es kann dem thermischen Verfahren bereits erfindungsgemäß behandelt zugeführt werden). Selbstverständlich kann die wenigstens eine Komplexverbindung aber auch erst im Verlauf des thermischen Trennverfahrens zugesetzt werden (z.B. bei einer Rektifikation in der Rücklaufflüssigkeit gelöst, oder bei einer Absorption im Absorptionsmittel gelöst, oder bei einer fraktionierenden Kondensation in der Rücklaufflüssigkeit gelöst, oder bei einer Direktkühlung des Produktgasgemisches der heterogen katalysierten partiellen Gasphasenoxidation der C₃-Vorläuferverbindung in der zur Direktkühlung eingesetzten Quenchflüssigkeit gelöst).

Selbstverständlich muss die der flüssigen Phase erfindungsgemäß zuzusetzende wenigstens eine Komplexverbindung nicht das einzige der flüssigen Phase zugesetzte Inhibitorsystem sein. Vielmehr kann die flüssige Phase zusätzlich einen oder mehrere Inhibitoren aus der Gruppe umfassend die Nitroxyl-Radikale (auch als N-Oxyl-Radikal bezeichnet) (z.B. die in der DE-A 19734171 offenbarten wie 4-Hydroxy-2,2,6,6-tetramethylpiperidin-1-oxyl oder 1,4-Dihydroxy-2,2,6,6,-tetramethylpiperidin), Phenothiazine wie z.B. Dibenzo-1,4-thiazin (Phenothiazin), phenolische Verbindungen wie Hydrochinon, 2,4-Dimethyl-6-t-butylphenol und Hydrochinonmonomethylether, molekularer Sauerstoff, p-Phenylendiamine (z.B. die in der DE-A 19734171 offenbarten), organische Nitrosoverbindungen wie 4-Nitrosophenol (und die anderen in der DE-A 19734171 offenbarten), Methylenblau und alle anderen z.B. in der EP-A 765856 offenbarten Inhibitoren zugesetzt enthalten. Vorgenannte Inhibitoren können der flüssigen Phase in entsprechenden Mengen zugesetzt sein, wie sie in dieser Schrift für die wenigstens eine der flüssigen Phase zuzusetzende Komplexverbindung ausgeführt sind und empfohlen werden.

Bei der Durchführung von nicht kristallisativen thermischen Trennverfahren an erfindungsgemäß behandelten flüssigen Phasen in trennwirksame Einbauten (z.B. Trennböden wie Dual-Flow-Böden) eingebaut enthaltenden Trennkolonnen kann als zusätzliche Inhibiermaßnahme als Quelle für molekularen Sauerstoff z.B. Luft oder mit Stickstoff angereicherte Luft (Magerluft) durch die Trennkolonne (z.B. eine Rektifikationskolonne oder Absorptionskolonne) geströmt werden, wie es z.B. in der DE-A 102009027401 und in der DE-A 102007004960 praktiziert wird.

Eine günstige Inhibitorkombination zur Stabilisierung der in der DE-A 102009027401 ausgeführten Rektifikation des Acrylsäure enthaltenden Absorbats A* in der Rektifikationskolonne K30 umfasst (neben der die Rektifikationkolonne K30 durchströmenden Luft), bezogen auf die jeweils zu stabilisierende (zu inhibierende) Menge an Acrylsäure, z.B. 0,1 bis 3 mol.-ppm Cu (zugesetzt in Form wenigstens einer Cu enthaltenden Verbindung (vorzugsweise Cu(II)-Di-n-butyldithiocarbamat) und 50 bis 1000 Gew.-ppm Phenothiazin, vorzugsweise 0,2 bis 2 mol.-ppm an Cu und 100 bis 500 Gew.-ppm Phenothiazin und besonders bevorzugt 0,3 bis 1 mol.-ppm Cu und 200 bis 400 Gew.-ppm Phenothiazin. Die Inhibitorzufuhr in die Rektifikationskolonne K30 erfolgt mit Vorteil über die Rücklaufflüssigkeit in selbiger gelöst, sowie über das der Rektifikationskolonne K30 zugeführte Absorbat A* in selbigem gelöst.

Korrosionsuntersuchungen haben ergeben, dass für erfindungsgemäß inhibierte flüssige Phasen, in denen das Metall Kupfer ist, der DIN Werkstoff 1.4571 ein geeigneter und in voll befriedigender Weise korrosionsbeständiger Apparatewerkstoff ist.

Die Erfindung wird durch die folgenden Beispiele näher erläutert, ohne sie dadurch einzuschränken.

### Beispiele

### 1. Herstellung von mit unterschiedlichen Polymerisationsinhibitoren versetzten flüssigen Phasen sowie von unterschiedlich inhibierten flüssigen Vergleichsphasen.

Wie in der DE-A 10 2007 055 086 beschrieben frisch hergestellte Reinacrylsäure, die, bezogen auf ihr Gewicht, mit 200 Gew.-ppm Methoxyphenol (MEHQ) polymerisationsinhibiert war, wurde unter vermindertem Druck (1000 Pa) durch zweifaches, aufeinanderfolgend durchgeführtes Überdestillieren von MEHQ befreit.

Die Reinheit des so erzeugten Reinacrylsäuredestillats betrug > 99,8 Gew.-%, bei einem Aldehyd- und Ketongesamtgehalt < 5 Gew.-ppm, einem Diacrylsäuregehalt < 1 Gew.-ppm.

Aus dem Reinacrylsäuredestillat wurde eine Teilmenge in identische Proben von 1 ml aufgeteilt.

Aus einer anderen Teilmenge wurden verschiedene Stammlösungen erzeugt, in welchen z.B. unterschiedliche Mengen unterschiedlicher Polymerisationsinhibitoren gelöst wurden.

Aus den Stammlösungen entnommene Probemengen wurden mit dem Reinacryldestillat in der jeweils erforderlichen Menge verdünnt und mit den Verdünnungen die verschiedenen 1 ml Proben wie gewünscht dotiert. Anschließend wurden die dotierten Proben durch Einfrieren konserviert.

### 2. Herstellung von Cu(II)oxinat aus verschiedenen Kupfersalzen

Es wurden Mischungen von 8-Hydroxychinolin mit diversen Kupferverbindungen hergestellt und mittels Dünnschichtchromatographie (DC) untersucht.

| | |
|---|---|
| DC-Laufmittel: | Acetonitril: 0,05m KH₂PO₄ (pH3,0) 6:4 |
| DC-Material: | TLC Silica gel 60 RP-18 F254 |

Folgende Kupferverbindungen wurden verwendet:
1. Kupfer(II)acetat Monohydrat
2. Kupfer(I)chlorid
3. Kupfer(II)-2,4-pentandionat
4. Kupfer(II)acetylacetonat
5. Cu(I)acetat
6. Cu(II)gluconat

### Durchführung:

Es wurde eine 8mmol/L Lösung an 8-Hydoxychinolin in Acrylsäure hergestellt.

Analog wurde von den Kupferverbindungen Lösungen mit einer Konzentration von 4mmol/L hergestellt. Jeweils 0,5mL der 8-Hydroxychinolin-Lösung wurden mit 0,5mL Kupfersalzlösung versetzt und gemischt. Diese Lösungen wurden mittels DC untersucht. Bei allen verwendeten Verbindungen wurde das gewünschte Kupfer(II)oxin gebildet.

### 3. Untersuchung der Polymerisationsneigung von Proben verschiedener flüssiger Phasen

Zur Untersuchung der Polymerisationsneigung der jeweiligen dotierten Probe wurde diese wieder verflüssigt und jeweils ein HPLC-Vial (transparentes Gefäß mit 1,5 ml Füllvolumen) mit 0,5 ml der jeweiligen Probe unter Luft befüllt und anschließend mit einer Bördelkappe dicht verschlossen. Unmittelbar nach Fertigstellung wurden jeweils bis zu sechs wie beschrieben befüllte Vials in eine dafür angefertigte Halterung eingehängt und bei einer Temperatur von 120°C in einem Umlufttrockenschrank sich selbst überlassen, während die Halterung mit sechs Umdrehungen pro Minute rotierte, um eine vollständige Durchmischung in den Vials zu gewährleisten (sechsmal pro Minute kam der flüssige Inhalt des jeweiligen Vials mit der Bördelkappe in Berührung). Dann wurde die Zeit T bis zur vollständigen Polymerisation der jeweiligen Probe im zugehörigen Vial erfasst. Dazu wurden die Proben in den Vials im Trockenschrank mit Hilfe einer Videokamera (die maximale Filmlaufzeit war 720 Minuten) überwacht und der Videofilm nachträglich visuell ausgewertet.

Jeder Versuch wurde dreimal wiederholt und die zugehörigen Werte für T arithmetisch gemittelt. Die resultierenden Mittelwerte t (in Minuten) für die verschiedenen Proben, einschließlich ihrer zugehörigen relevanten Gehalte an von Acrylsäure verschiedenen Bestandteilen, sind nachfolgend aufgelistet (die Mengenangaben sind jeweils bezogen auf die in der jeweiligen Probe enthaltene Menge an Acrylsäure).

**Tabelle 1**

| Stabilisierung von Acrylsäure mit Cu(II)acetat und verschiedenen Liganden | | |
|---|---|---|
| Ligand | Cu-Salz | t** |
| ohne Stabilisator | | 24 |
| 0,05mmol/L Phenothiazin | | 144 |
| 0,05mmol/L Cu(II)acetat* | | 47 |
| 0,10mmol/L 8-Hydroxychinolin | | 30 |
| 0,10mmol/L 8-Hydroxy-5-methylchinolin | | 29 |
| 0,1 0mmol/L 5,7-Dibrom-8-(benzoyloxy)-2-methylchinolin | | 29 |
| 0,10mmol/L Propionsäure-5-chlor-chinolin-8-ylester | | 34 |
| 0,10mmol/L 8-Acetoxychinolin | | 31 |
| 0,10mmol/L 8-Hydroxychinolin-5-sulfonsäure | | 29 |
| 0,10mmol/L 8-Aminochinolin | | 24 |
| 0,10mmol/L 8-Aminochinaldin | | 22 |
| 0,10mmol/L 8-Amino-5-Chloro-6-methoxychinolin | | 23 |
| 0,10mmol/L 8-Hydroxychinolin-N-Oxid | | 33 |
| 0,10mmol/L 5-Amino-8-Hydroxychinolin | | 22 |
| 0,10mmol/L Lupragen® N205 | | 25 |
| 0,10mmol/L Lupragen® N301 | | 23 |
| 0,10mmol/L Lupragen® N600 | | 24 |
| 0,10mmol/L 1,1,4,7,10,10-Hexamethylentriethylentetramin | | 20 |
| 0,10mmol/L Indigo | | 33 |
| 0,05mmol/L Phenothiazin | + 0,05mmol/L Cu(II)acetat* | 147 |
| 0,10mmol/L 8-Hydroxychinolin | + 0,05mmol/L Cu(II)acetat* | >700 |
| 0,10mmol/L 8-Hydroxy-5-methylchinolin | + 0,05mmol/L Cu(II)acetat* | 504 |
| 0,10mmol/L 5,7-Dibrom-8-(benzoyloxy)-2-methylchinolin | + 0,05mmol/L Cu(II)acetat* | 284 |
| 0,10mmol/L Propionsäure-5-chlor-chinolin-8-ylester | + 0,05mmol/L Cu(II)acetat* | 529 |
| 0,10mmol/L 5-Chlor-8-Hydroxychinolin | + 0,05mmol/L Cu(II)acetat* | >700 |
| 0,10mmol/L 5,7-Dibrom-8-hydroxy-2-methylchinolin | + 0,05mmol/L Cu(II)acetat* | 301 |
| 0,10mmol/L 8-Acetoxychinolin | + 0,05mmol/L Cu(II)acetat* | 590 |
| 0,10mmol/L 8-Hydroxychinolin-5-sulfonsäure | + 0,05mmol/L Cu(II)acetat* | 418 |
| 0,10mmol/L 8-Aminochinolin | + 0,05mmol/L Cu(II)acetat* | 538 |
| 0,10mmol/L 8-Aminochinaldin | + 0,05mmol/L Cu(II)acetat* | 531 |
| 0,10mmol/L 8-Amino-5-Chloro-6-methoxychinolin | + 0,05mmol/L Cu(II)acetat* | 198 |
| 0,10mmol/L 8-Hydroxychinolin-N-Oxid | + 0,05mmol/L Cu(II)acetat* | >700 |
| 0,10mmol/L 5-Amino-8-Hydroxychinolin | + 0,05mmol/L Cu(II)acetat* | >700 |
| 0,10mmol/L Lupragen® N205 | + 0,05mmol/L Cu(II)acetat* | 213 |
| 0,10mmol/L Lupragen® N301 | + 0,05mmol/L Cu(II)acetat* | 253 |
| 0,10mmol/L Lupragen® N600 | + 0,05mmol/L Cu(II)acetat* | 524 |
| 0,10mmol/L 1,1,4,7,10,10-Hexamethylentriethylentetramin | + 0,05mmol/L Cu(II)acetat* | 436 |
| 0,10mmol/L Indigo | + 0,05mmol/L Cu(II)acetat* | 397 |

| | | |
|---|---|---|
| * als Monohydrat ** t bezeichnet die Zeit in Minuten bis Polymerisation | | |

**Tabelle 2**

| Stabilisierung von Acrylsäure mit 8-Hydroxychinolinkomplexen verschiedener Metallsalze | | |
|---|---|---|
| Metallsalz | Ligand | t** |
| | 0,05mmol/L Phenothiazin | 143 |
| | 0,10mmo/L 8-Hydroxychinolin | 30 |
| 0,05mmol/L Cu(I)acetat | | 51 |
| 0,05mmol/L Cu(II)acetat* | | 44 |
| 0,05mmol/L Mn(II)acetat | | 429 |
| 0,05mmol/L Mn(III)acetat* | | 25 |
| 0,05mmol/L Co(II)acetat | | 21 |
| 0,05mmol/L Ce(III)acetat* | | 430 |
| 0,05mmol/L Ni(II)acetylacetonat | | 28 |
| 0,05mmol/L Cu(I)acetat | + 0,10mmol/L 8-Hydroxychinolin | 461 |
| 0,05mmol/L Cu(II)acetat* | + 0,10mmol/L 8-Hydroxychinolin | >700 |
| 0,05mmol/L Mn(II)acetat | + 0,10mmol/L 8-Hydroxychinolin | >700 |
| 0,05mmol/L Mn(III)acetat* | + 0,10mmol/L 8-Hydroxychinolin | >700 |
| 0,05mmol/L Ce(III)acetat* | + 0,10mmol/L 8-Hydroxychinolin | >700 |

| | | |
|---|---|---|
| * als Monohydrat ** t bezeichnet die Zeit in Minuten bis Polymerisation | | |

**Tabelle 3**

| Abhängigkeit der Stabilisierung von Acrylsäure vom Molverhältnis Cu(II) zu Liganden | | | |
|---|---|---|---|
| Cu(II)acetat* in Konzentration mmol/l | Konzentration [mmol/l] / Ligand | | t** |
| 0,025 | 0,050 | 8-Hydroxychinolin | 244 |
| 0,025 | 0,100 | 8-Hydroxychinolin | 287 |
| 0,050 | 0,100 | 8-Hydroxychinolin | >700 |
| 0,025 | 0,200 | 8-Hydroxychinolin | 490 |
| 0,050 | 0,200 | 8-Hydroxychinolin | >700 |
| 0,025 | 0,050 | Indigo | 61 |
| 0,025 | 0,100 | Indigo | 101 |
| 0,050 | 0,100 | Indigo | 360 |
| 0,025 | 0,200 | Indigo | 511 |
| 0,050 | 0,200 | Indigo | >700 |
| 0,050 | 0,050 | Lupragen® N 205 | 101 |
| 0,050 | 0,100 | Lupragen® N 205 | 213 |
| 0,050 | 0,200 | Lupragen® N 205 | 530 |
| 0,025 | 0,050 | Lupragen® N 301 | 76 |
| 0,025 | 0,100 | Lupragen® N 301 | 143 |
| 0,050 | 0,100 | Lupragen® N 301 | 253 |
| 0,025 | 0,200 | Lupragen® N 301 | 298 |
| 0,050 | 0,200 | Lupragen® N 301 | 552 |
| 0,050 | 0,400 | Lupragen® N 301 | >700 |
| 0,050 | 0,050 | Lupragen® N 600 | 148 |
| 0,050 | 0,100 | Lupragen® N 600 | 524 |
| 0,050 | 0,200 | Lupragen® N 600 | >700 |
| 0,050 | 0,050 | 1,1,4,7,10,10-Hexamethyltriethylentetramin | 148 |
| 0,050 | 0,100 | 1,1,4,7,10,10-Hexamethyltriethylentetramin | 524 |
| 0,050 | 0,200 | 1,1,4,7,10,10-Hexamethyltriethylentetramin | >700 |

| | | | |
|---|---|---|---|
| * als Monohydrat ** t bezeichnet die Zeit in Minuten bis Polymerisation | | | |

**Tabelle 4**

| Stabilisierung von Methacrylsäure | |
|---|---|
| Stabilisator | t** |
| 0,050mmol/L Phenothiazin | 35 |
| 0,050 mmol/l Cu(II)dibutyldithiocarbamat | 205 |
| 0,050 mmol/l Cu(II)oxin | > 700 |
| 0,050 mmol/l Cu(II)acetat* + 0100mmol/L 8-Aminochinolin | >700 |
| 0,050mmol/L Cu(II)acetat* + 0,200mmol/L 1,1,4,7,10,10-Hexamethyltriethylentetramin | >700 |
| 0,050mmol/L Cu(II)acetat* + 0,200mmol/L Lupragen® N205 | >700 |
| 0,050mmol/L Cu(II)acetat* + 0,200mmol/L Lupragen® N600 | >700 |

| | |
|---|---|
| * als Monohydrat ** t bezeichnet die Zeit in Minuten bis zur Polymerisation | |

**Tabelle 5**

| Stabilisierung von Acrylsäureestern | | |
|---|---|---|
| Acrylsäureester | Stabilisator | t** |
| 2-Ethylhexylacrylat | 0,050 mmol/l Cu(II)oxin | 364 |
| 2-Ethylhexylacrylat | 0,050 mmol/l Cu(II)acetat* + 0,100mmol/l 8 Aminochinolin | >700 |
| 2-Ethylhexylacrylat | 0,050mmol/L Cu(II)acetat* + 0,200mmol/L 1,1,4,7,10,10-Hexamethyltriethylentetramin | 122 |
| 2-Ethylhexylacrylat | 0,050mmol/L Cu(II)acetat* + 0,200mmol/L Lupragen® N205 | 112 |
| 2-Ethylhexylacrylat | 0,050mmol/L Cu(II)acetat* + 0,200mmol/L Lupragen® N600 | 619 |
| Ethylacrylat | 0,050 mmol/l Cu(II)oxin | >700 |
| Ethylacrylat | 0,050 mmol/l Cu(II)acetat* + 0,100mmol/l 8 Aminochinolin | >700 |
| Ethylacrylat | 0,050mmol/L Cu(II)acetat* + 0,200mmol/L 1,1,4,7,10,10-Hexamethyltriethylentetramin | >700 |
| Ethylacrylat | 0,050mmol/L Cu(II)acetat* + 0,200mmol/L Lupragen® N205 | >700 |
| Ethylacrylat | 0,050mmol/L Cu(II)acetat* + 0,200mmol/L Lupragen® N600 | >700 |
| Methylacrylat | 0,050 mmol/l Cu(II)oxin | >700 |
| Methylacrylat | 0,050 mmol/l Cu(II)acetat* + 0,100mmol/l 8 Aminochinolin | >700 |
| Methylacrylat | 0,050mmol/L Cu(II)acetat* + 0,200mmol/L 1,1,4,7,10,10-Hexamethyltriethylentetramin | >700 |
| Methylacrylat | 0,050mmol/L Cu(II)acetat* + 0,200mmol/L Lupragen® N205 | >700 |
| Methylacrylat | 0,050mmol/L Cu(II)acetat* + 0,200mmol/L Lupragen® N600 | >700 |
| n-Butylacrylat | 0,050 mmol/l Cu(II)oxin | >700 |
| n-Butylacrylat | 0,050 mmol/l Cu(II)acetat* + 0,100mmol/l 8 Aminochinolin | >700 |
| tert.-Butylacrylat | 0,050 mmol/l Cu(II)oxin | >700 |
| tert.-Butylacrylat | 0,050 mmol/l Cu(II)acetat* + 0,100mmol/l 8 Aminochinolin | >700 |

| | | |
|---|---|---|
| * als Monohydrat ** t bezeichnet die Zeit in Minuten bis zur Polymerisation | | |

## Patentansprüche

1. Verfahren zur Stabilisierung von Acrylverbindungen, wobei man eine flüssige Phase, welche mindestens eine Acrylverbindung aus der Gruppe Acrylsäure, Methacrylsäure und deren jeweiligen Estern enthält, mit mindestens einem Metall aus der Gruppe Kupfer, Mangan und Cer versetzt sowie mindestens einem Liganden aus der Gruppe bestehend aus
a) Chinolinverbindungen der Formel (I), 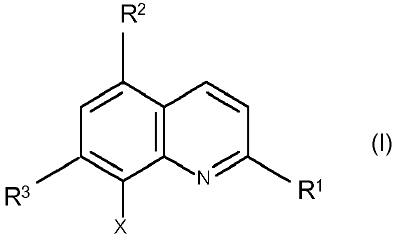 wobei die Symbole folgende Bedeutungen haben:
X ist OH, NH₂, O-C₁-C₄-Alkyl, vorzugsweise OCH₃, O-C(O)-C₁-C₄-Alkyl, vorzugsweise O-C(O)-CH₃, O-C(O)-C₂H5 oder O-C(O)-Phenyl;
R¹ ist H, oder (C₁-C₄)-Alkyl, vorzugsweise Methyl;
R² ist H, C₁-C₄-Alkyl, vorzugsweise Methyl, Cl, Br oder SO₃H und
R³ ist H, Cl oder Br;
sowie N-Oxiden von Verbindungen der Formel (I),
b) 2,2'-Bis(2,3-dihydro-3-oxoindolyliden) und
c) aliphatische y-zähnige Liganden mit y = 2 - 6, enthaltend mindestens zwei Stickstoffatome und y-2 weitere koordinierende Stickstoff- oder Heteroatome enthaltende aliphatische oder aromatische C₁-C₄ Kohlenstoffbrücken verbunden sind, ausgewählt aus 1,1,4,7,10,10-Hexamethyltriethylentetramin, N.N.N',N",N"-Pentamethyl-diethylentriamin, N,N,N',N'-Tetramethylethan-1,2-diamin, N,N,N',N'-Tetramethylpropan-1,3-diamin, 1,3,5-Tris(dimethylaminopropyl)-sym-hexahydrotriazin, Bis(2-dimethylaminoethyl)ether und Tris[2-(dimethylamino)ethyl]amin.

2. Verfahren gemäß Anspruch 1, **dadurch gekennzeichnet, dass** der flüssigen Phase mindestens ein Salz des mindestens einen Metalls und mindestens ein Ligand zugesetzt werden und sich in der flüssigen Phase ein Komplex bildet.

3. Verfahren gemäß Anspruch 1 oder 2, **dadurch gekennzeichnet, dass** das Metall Kupfer ist.

4. Verfahren gemäß Anspruch 3, **dadurch gekennzeichnet, dass** das Metall Kupfer(II) ist.

5. Verfahren gemäß einem der Ansprüche 2 bis 4, **dadurch gekennzeichnet, dass** Kupfer(II)acetat als Metallsalz eingesetzt wird.

6. Verfahren gemäß einem der Ansprüche 1 bis 5, **dadurch gekennzeichnet, dass** der Gehalt der flüssigen Phase an Metall 0,01 mol bis 5 mol-%, bezogen auf die Acrylverbindung, beträgt.

7. Verfahren gemäß einem der Ansprüche 1 bis 6, **dadurch gekennzeichnet, dass** der Ligand aus der Gruppe a) ausgewählt wird aus 8-Hydroxychinolin, 8-Hydroxy-5-methylchinolin, 5-Chlorchinolin-8-yl-propionat, 5-Chlor-8-hydroxychinolin, 5,7-Dibrom-8-hydroxy-2-methylchinolin, 8-Hydroxy-2-methylchinolin (8-Hydrocychinaldin), 8-Acetoxychinolin, 8-Aminochinolin, 8-Amino-2-methylchinolin (8-Aminochinaldin) und 5-Amino-8-hydroxychinolin, 8-Hydroxychinolin-N-Oxid.

8. Verfahren gemäß Anspruch 7, wobei der Ligand 8-Hydroxychinolin ist.

9. Verfahren gemäß einem der Ansprüche 1 bis 8, wobei das molekulare Verhältnis von Metall zu Ligand von 1:1 bis 1:10 beträgt.

10. Verfahren gemäß einem der Ansprüche 1 bis 9, wobei die Acrylverbindung Acrylsäure ist.

11. Flüssige Phase, enthaltend mindestens 10 Gew.-% an mindestens einer Verbindung aus der Gruppe Acrylsäure, Methacrylsäure und deren jeweiligen Estern und mindestens ein Metall aus der Gruppe Kupfer, Mangan und Cer sowie mindestens einen Liganden aus der Gruppe bestehend aus a) Chinolinverbindungen der Formel (I) gemäß Anspruch 1 sowie N-Oxiden von Verbindungen der Formel (I), b) 2,2'-Bis(2,3-dihydro-3-oxoindolyliden), und c) aliphatischen y-zähnigen Liganden mit y = 2 - 6, enthaltend mindestens zwei Stickstoffatome und y-2 weitere koordinierende Stickstoff- oder Heteroatome enthaltende aliphatische oder aromatische C₁-C₄ Kohlenstoffbrücken verbunden sind, ausgewählt aus 1,1,4,7,10,10-Hexamethyltriethylentetramin, N.N.N',N",N"-Pentamethyl-diethylentriamin, N,N,N',N'-Tetramethylethan-1,2-diamin, N,N,N',N'-Tetramethylpropan-1,3-diamin, 1,3,5-Tris(dimethylaminopropyl)-sym-hexahydrotriazin, Bis(2-dimethylaminoethyl)ether und Tris[2-(dimethylamino)ethyl]amin.

12. Verwendung eines Komplexes, enthaltend mindestens ein Metall aus der Gruppe Kupfer, Mangan und Cer sowie mindestens einen Liganden aus der Gruppe bestehend aus a) Chinolinverbindungen der Formel (I) sowie N-Oxiden von Verbindungen der Formel (I) gemäß Anspruch 1, b) 2,2'-Bis(2,3-dihydro-3-oxoindolyliden) und c) aliphatischen y-zähnigen Liganden mit y = 2 - 6, enthaltend mindestens zwei Stickstoffatome und y-2 weitere koordinierende Stickstoff- oder Heteroatome enthaltende aliphatische oder aromatische C₁-C₄ Kohlenstoffbrücken verbunden sind, ausgewählt aus 1,1,4,7,10,10-Hexamethyltriethylentetramin, N.N.N',N",N"-Pentamethyl-diethylentriamin, N,N,N',N'-Tetramethylethan-1,2-diamin, N,N,N',N'-Tetramethylpropan-1,3-diamin, 1,3,5-Tris(dimethylaminopropyl)-sym-hexahydrotriazin, Bis(2-dimethylaminoethyl)ether und Tris[2-(dimethylamino)ethyl]amin, als Polymerisationsinhibitor zur Stabilisierung von Acrylsäure, Methacrylsäure und/oder deren jeweiligen Estern.

## Claims

1. A method of stabilizing acrylic compounds, wherein a liquid phase comprising at least one acrylic compound selected from the group consisting of acrylic acid, methacrylic acid and the respective esters thereof is admixed with at least one metal selected from the group consisting of copper, manganese and cerium and also at least one ligand selected from the group consisting of
a) quinoline compounds of the formula (I) 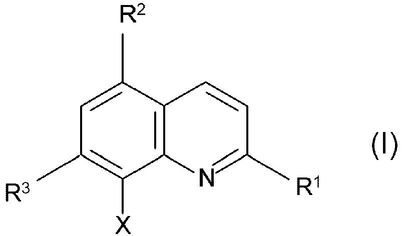 where the symbols have the following meanings
X is OH, NH₂, O-C₁-C₄-alkyl, preferably OCH₃, O-C(O)-C₁-C₄-alkyl, preferably O-C(O)-CH₃, O-C(O)-C₂H₅ or O-C(O)-phenyl;
R¹ is H, or (C₁-C₄)-alkyl, preferably methyl;
R² is H, C₁-C₄-alkyl, preferably methyl, Cl, Br or SO₃H and
R³ is H, Cl or Br
and also N-oxides of compounds of the formula (I),
b) 2,2'-bis(2,3-dihydro-3-oxoindolylidene) and
c) aliphatic y-dentate ligands having y = 2 - 6 and comprising at least two nitrogen atoms joined by aliphatic or aromatic C₁-C₄ bridges comprising y-2 further coordinating nitrogen atoms or heteroatoms, selected from among 1,1,4,7,10,10-hexamethyltriethylenetetramine, N,N,N',N",N"-pentamethyldiethylenetriamine, N,N,N',N'-tetramethylethane-1,2-diamine, N,N,N',N'-tetramethylpropane-1,3-diamine, 1,3,5-tris(dimethylaminopropyl)sym-hexahydrotriazine, bis(2-dimethylaminoethyl) ether and tris[2-(dimethylamino)ethyl] amine

2. The method according to claim 1, wherein at least one salt of the at least one metal and at least one ligand are added to the liquid phase and a complex is formed in the liquid phase

3. The method according to claim 1 or 2, wherein the metal is copper.

4. The method according to claim 3, wherein the metal is copper(II).

5. The method according to any of claims 2 to 4, wherein copper(II) acetate is used as metal salt.

6. The method according to any of claims 1 to 5, wherein the content of metal in the liquid phase is from 0.01 mol to 5 mol%, based on the acrylic compound.

7. The method according to any of claims 1 to 6, wherein the ligand of group a) is selected from among 8-hydroxyquinoline, 8-hydroxy-5-methylquinoline, 5-chloroquinoline-8-yl propionate, 5-chloro-8-hydroxyquinoline, 5,7-dibromo-8-hydroxy-2-methylquinoline, 8-hydroxy-2-methylquinoline (8-hydroxyquinaldine), 8-acetoxyquinoline, 8-aminoquinoline, 8-amino-2-methylquinoline (8-aminoquinaldine), 5-amino-8-hydroxyquinoline and 8-hydroxyquinoline N-oxide.

8. The method according to claim 7, wherein the ligand is 8-hydroxyquinoline.

9. The method according to any of claims 1 to 8, wherein the molar ratio of metal to ligand is from 1:1 to 1:10.

10. The method according to any of claims 1 to 9, wherein the acrylic compound is acrylic acid.

11. A liquid phase comprising at least 10% by weight of at least one compound selected from the group consisting of acrylic acid, methacrylic acid and the respective esters thereof and at least one metal selected from the group consisting of copper, manganese and cerium and also at least one ligand selected from the group consisting of a) quinoline compounds of the formula (I) according to claim 1 and also N-oxides of compounds of the formula (I), b) 2,2'-bis(2,3-dihydro-3-oxoindolylidene), and c) aliphatic y-dentate ligands having y = 2 - 6 and comprising at least two nitrogen atoms joined by aliphatic or aromatic C₁-C₄ bridges comprising y-2 further coordinating nitrogen atoms or heteroatoms, selected from among 1,1,4,7,10,10-hexamethyltriethylenetetramine, N,N,N',N",N"-pentamethyldiethylenetriamine, N,N,N',N'-tetramethylethane-1,2-diamine, N,N,N',N'-tetramethylpropane-1,3-diamine, 1,3,5-tris(dimethylaminopropyl)sym-hexahydrotriazine, bis(2-dimethylaminoethyl) ether and tris[2-(dimethylamino)ethyl]amine

12. The use of a complex comprising at least one metal selected from the group consisting of copper, manganese and cerium and at least one ligand selected from the group consisting of a) quinoline compounds of the formula (I) and also N-oxides of compounds of the formula (I) according to claim 1, b) 2,2'-bis(2,3-dihydro-3-oxoindolylidene), and c) aliphatic y-dentate ligands having y = 2 - 6 and comprising at least two nitrogen atoms joined by aliphatic or aromatic C₁-C₄ bridges comprising y-2 further coordinating nitrogen atoms or heteroatoms, selected from among 1,1,4,7,10,10-hexamethyltriethylenetetramine, N,N,N',N",-pentamethyldiethylenetriamine, N, N, N', N'-tetramethylethane-1,2-diamine, N,N,N',N'-tetramethylpropane-1,3-diamine, 1,3,5-tris(dimethylaminopropyl)sym-hexahydrotriazine, bis(2-dimethylaminoethyl) ether and tris[2-(dimethylamino)ethyl]amine, as polymerization inhibitor for stabilizing acrylic acid, methacrylic acid and/or the respective esters thereof.

## Revendications

1. Procédé de stabilisation de composés acryliques, selon lequel une phase liquide, qui contient au moins un compose acrylique du groupe constitué par l'acide acrylique, l'acide méthacrylique et leurs esters respectifs, est mélangée avec au moins un métal du groupe constitué par le cuivre, le manganèse et le cérium, ainsi qu'au moins un ligand du groupe constitué par :
a) les composés de quinoline de formule (I) 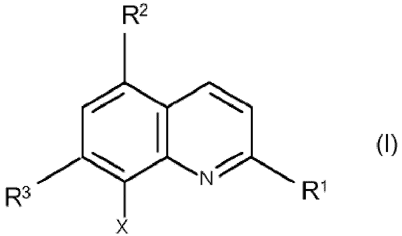 dans laquelle les symboles ont les significations suivantes :
X représente OH, NH₂, O-alkyle en C₁-C₄, de préférence OCH₃, O-C(O)-alkyle en C₁-C₄, de préférence O-CO)-CH₃, O-C(O)-C₂H₅ ou O-C(O)-phényle ;
R¹ représente H ou alkyle en (C₁-C₄), de préférence méthyle ;
R² représente H, alkyle en C₁-C₄, de préférence méthyle, Cl, Br ou SO₃H, et
R³ représente H, Cl, ou Br ;
ainsi que les N-oxydes de composés de formule (I),
b) le 2,2'-bis(2,3-dihydro-3-oxoindolylidène) et
c) les ligands aliphatiques y-dentates avec y = 2 à 6, contenant au moins deux atomes d'azote et y-2 ponts carbonés en C₁-C₄ aliphatiques ou aromatiques contenant des atomes d'azote ou hétéroatomes coordinants supplémentaires sont connectés, choisis parmi la 1,1,4,7,10,10-hexaméthyltriéthylène-tétramine, la N,N,N',N",,N"-pentaméthyl-diéthylène-triamine, la N,N,N',N'-tétraméthyléthane-1,2-diamine, la N,N,N',N'-tétraméthylpropane-1,3-diamine, la 1,3,5-tris(diméthylaminopropyl)-sym-hexahydrotriazine, l'éther bis(2-diméthylaminoéthylique) et la tris[2-(diméthylamino)éthyl]amine.

2. Procédé selon la revendication 1, **caractérisé en ce qu'**au moins un sel dudit au moins un métal et au moins un ligand sont ajoutés à la phase liquide, et un complexe se forme dans la phase liquide.

3. Procédé selon la revendication 1 ou 2, **caractérisé en ce que** le métal est le cuivre.

4. Procédé selon la revendication 3, **caractérisé en ce que** le métal est le cuivre (II).

5. Procédé selon l'une quelconque des revendications 2 à 4, **caractérisé en ce que** l'acétate de cuivre (II) est utilisé en tant que sel métallique.

6. Procédé selon l'une quelconque des revendications 1 à 5, **caractérisé en ce que** la teneur de la phase liquide en métal est de 0,01 à 5 % en moles, par rapport au composé acrylique.

7. Procédé selon l'une quelconque des revendications 1 à 6, **caractérisé en ce que** le ligand du groupe a) est choisi parmi la 8-hydroxyquinoline, la 8-hydroxy-5-méthylquinoline, le propionate de 5-chloroquinolin-8-yle, la 5-chloro-8-hydroxyquinoline, la 5,7-dibromo-8-hydroxy-2-méthyl-quinoline, la 8-hydroxy-2-méthylquinoline (8-hydroxyquinaldine), la 8-acétoxyquinoline, la 8-amino-quinoline, la 8-amino-2-méthylquinoline (8-aminoquinaldine) et la 5-amino-8-hydroxyquinoline, le 8-hydroxyquinoline-N-oxyde.

8. Procédé selon la revendication 7, dans lequel le ligand est la 8-hydroxyquinoline.

9. Procédé selon l'une quelconque des revendications 1 à 8, dans lequel le rapport moléculaire entre le métal et le ligand est de 1:1 à 1:10.

10. Procédé selon l'une quelconque des revendications 1 à 9, dans lequel le composé acrylique est l'acide acrylique.

11. Phase liquide, contenant au moins 10 % en poids d'au moins un composé du groupe constitué par l'acide acrylique, l'acide méthacrylique et leurs esters respectifs, et au moins un métal du groupe constitué par le cuivre, le manganèse et le cérium, ainsi qu'au moins un ligand du groupe constitué par a) les composés de quinoline de formule (I) selon la revendication 1, ainsi que les N-oxydes de composés de formule (I), b) le 2,2'-bis(2,3-dihydro-3-oxoindolylidène) et c) les ligands aliphatiques y-dentates avec y = 2 à 6, contenant au moins deux atomes d'azote et y-2 ponts carbonés en C₁-C₄ aliphatiques ou aromatiques contenant des atomes d'azote ou hétéroatomes coordinants supplémentaires sont connectés, choisis parmi la 1,1,4,7,10,10-hexaméthyltriéthylène-tétramine, la N,N,N',N'',N''-pentaméthyl-diéthylène-triamine, la N,N,N',N'-tétraméthyléthane-1,2-diamine, la N,N,N',N' tétraméthylpropane-1,3-diamine, la 1,3,5-tris(diméthylaminopropyl)-sym-hexahydrotriazine, l'éther bis(2-diméthylaminoéthylique) et la tris[2-(diméthylamino)éthyl]amine.

12. Utilisation d'un complexe, contenant au moins un métal du groupe constitué par le cuivre, le manganèse et le cérium, ainsi qu'au moins un ligand du groupe constitué par a) les composés de quinoline de formule (I), ainsi que les N-oxydes de composés de formule (I) selon la revendication 1, b) le 2,2'-bis(2,3-dihydro-3-oxoindolylidène) et c) les ligands aliphatiques y-dentates avec y = 2 à 6, contenant au moins deux atomes d'azote et y-2 ponts carbonés en C₁-C₄ aliphatiques ou aromatiques contenant des atomes d'azote ou hétéroatomes coordinants supplémentaires sont connectés, choisis parmi la 1,1,4,7,10,10-hexaméthyltriéthylène-tétramine, la N,N,N',N",N"-pentaméthyl-diéthylène-triamine, la N,N,N',N'-tétraméthyléthane-1,2-diamine, la N,N,N',N'-tétraméthylpropane-1,3-diamine, la 1,3,5-tris(diméthylaminopropyl)-sym-hexahydrotriazine, l'éther bis(2-diméthylaminoéthylique) et la tris[2-(diméthylamino)éthyl]aminé, en tant qu'inhibiteur de polymérisation pour la stabilisation d'acide acrylique, d'acide méthacrylique et/ou leurs esters respectifs.
